# EUROPEAN PATENT SPECIFICATION

(11) **EP 1 448 230 B1**
(45) Date of publication and mention of the grant of the patent: **20.10.2010**
(21) Application number: 02788442.8
(22) Date of filing: 04.12.2002
(51) Int. Cl.: A61K 39/39, A61K 39/116, A61K 39/295, A61P 31/04

(54) **ADJUVANTED ANTIGENIC MENINGOCOCCAL COMPOSITIONS**
ADJUVIERTE ANTIGENE MENINGOKOKKENZUSAMMENSETZUNGEN
COMPOSITIONS MENINGOCOCCIQUES ANTIGENIQUES AVEC ADJUVANTS

(30) Priority: 04.12.2001 GB 0129007
(43) Date of publication of application: 25.08.2004
(73) Proprietor: Novartis Vaccines and Diagnostics S.r.l., 53100 Siena (SI) (IT)
(72) Inventor: PIZZA, Mariagrazia,Chiron S.p.A., I-53100 Siena (IT); GIULIANI, Marzia, Monica, Chiron SpA, I-53100 Siena (IT)
(74) Representative: Marshall, Cameron John
(86) International application number: PCT/IB2002/005662
(87) International publication number: WO 2003/047619

(56) References cited:
- WO-A-00/18434
- WO-A-99/24578
- WO-A-99/36544
- PIZZA M ET AL: "Mucosal vaccines: non toxic derivatives of LT and CT as mucosal adjuvants" VACCINE, BUTTERWORTH SCIENTIFIC. GUILDFORD, GB, vol. 19, no. 17-19, 21 March 2001 (2001-03-21), pages 2534-2541, XP004231077 ISSN: 0264-410X
- DALSEG R ET AL: "Outer membrane vesicles from group B meningococci are strongly immunogenic when given intranasally to mice" VACCINE, BUTTERWORTH SCIENTIFIC. GUILDFORD, GB, vol. 17, no. 19, 14 May 1999 (1999-05-14), pages 2336-2345, XP004168964 ISSN: 0264-410X
- BARTOLONI A ET AL: "Immunogenicity of meningococcal B polysaccharide conjugated to tetanus toxoid or CRM197 via adipic acid dihydrazide" VACCINE, BUTTERWORTH SCIENTIFIC. GUILDFORD, GB, vol. 13, no. 5, 1995, pages 463-470, XP004057721 ISSN: 0264-410X

## Description

### TECHNICAL FIELD

This invention is in the field of vaccines, and in particular of vaccines against bacteria from the *Neisseria* genus (*e.g. N.gonorrhoeae* or, preferably, *N.meningitidis*).

### BACKGROUND ART

References 1 to 6 disclose antigens, proteins and open reading frames from *Neisseria* bacteria, including *N.gonorrhoeae* and serogroups A and B of *N.meningitidis.* References 7 to 9 disclose various ways of expressing these proteins. Reference 10 discloses the enhancement of immunogenicity of *Neisseria* antigens when CpG adjuvants are used as adjuvants.

It is an object of the present invention to provide alternative and improved ways of enhancing immune responses raised against antigens from *Neisseria,* particularly *N.meningitidis* serogroup B. WO 9936544 discloses the sequences of Neisserial proteins including ORF40.

### DISCLOSURE OF THE INVENTION

The invention provides a composition comprising an ORF40 Neisserial antigen and the detoxified ADP-ribosylating toxin LT-R72. These compositions have been found to be useful for mucosal immunisation.

**ORF40 (SEQ ID NO: 4 of** WO9936544**)**

MNKIYRIIWNSALNAWVVVSELTRNHTKRASATVKTAVLATLLFATVQASA
NNEEQEEDLYLDPVQRTVAVLIVNSDKEGTGEKEKVEENSDWAVYFNEKGV
LTAREITLKAGDNLKIKQNGTNFTYSLKKDLTDLTSVGTEKLSFSANGNKVNI
TSDTKGLNFAKETAGTNGDTTVHLNGIGSTLTDTLLNTGATTNVTNDNVTDD
EKKRAASVKDVLNAGWNIKGVKPGTTASDNVDFVRTYDTVEFLSADTKTTT
VNVESKDNGKKTEVKIGAKTSVIKEKDGKLVTGKDKGENGSSTDEGEGLVT
AKEVIDAVNKAGWRMKTTTANGQTGQADKFETVTSGTNVTFASGKGTTAT
VSKDDQGNITVMYDVNVGDALNVNQLQNSGWNLDSKAVAGSSGKVISGNV
SPSKGKMDETVNINAGNNIEITRNGKNIDIATSMTPQFSSVSLGAGADAPTLSV
DGDALNVGSKKDNKPVRITNVAPGVKEGDVTNVAQLKGVAQNLNNRIDNV
DGNARAGIAQAIATAGLVQAYLPGKSMMAIGGGTYRGEAGYAIGYSSISDGG
NWIIKGTASGNSRGHFGASASVGYQW

The composition is preferably an immunogenic composition, and more preferably a vaccine.

### The Neisserial antigen

There are described compositions wherein the Neisserial antigen is preferably a *N.meningitidis* antigen, and more preferably a *N.meningitidis* serogroup B antigen. Within serogroup B, preferred strains are 2996, MC58, 95N477, or 394/98.

There are also described compositions wherein the Neisserial antigen is preferably a protein antigen. More preferably, the protein antigen is selected from the group consisting of:
(a) a protein comprising one or more of the 446 even SEQ IDs (*i.e.* 2, 4, 6, ... , 890, 892) disclosed in reference 1.
(b) a protein comprising one or more of the 45 even SEQ IDs (*i*.*e*. 2, 4, 6, ... , 88, 90) disclosed in reference 2;
(c) a protein comprising one or more of the 1674 even SEQ IDs 2-3020, even SEQ IDs 3040-3114, and all SEQ IDs 3115-3241 disclosed in reference 3;
(d) a protein comprising one or more of the 2160 amino acid sequences NMB0001 to NMB2160 disclosed in reference 5;
(e) a protein comprising an amino acid sequence having sequence identity to an amino acid sequence specified in (a), (b), (c) or (d);
(f) a protein comprising a fragment of an amino acid sequence specified in (a), (b), (c) or (d);
(g) a protein comprising one or more of the amino acid sequences disclosed in reference 7, reference 8 or reference 9; or
(h) a protein having formula NH₂-A-[-X-L-]*ₙ*-B-COOH, wherein X is an amino acid sequence, L is an optional linker amino acid sequence, A is an optional N-terminal amino acid sequence, B is an optional C-terminal amino acid sequence, and n is an integer greater than 1.

The degree of 'sequence identity' referred to in (e) may be greater than 50% *(eg.* 60%, 70%, 80%, 90%, 95%, 99% or more, up to 100%). This includes mutants, homologs, orthologs, allelic variants *etc*. [*e.g.* see reference 11]. Identity is preferably determined by the Smith-Waterman homology search algorithm as implemented in the MPSRCH program (Oxford Molecular), using an affine gap search with parameters *gap open penalty=12* and *gap extension penalty=1.* Typically, 50% identity or more between two proteins is considered to be an indication of functional equivalence.

The 'fragments' referred to in (f) may consist of least m consecutive amino acids from an amino acid sequence from (a), (b), (c), (d) or (e) and, depending on the particular sequence, *m* may be 7 or more *(eg.* 8, 10, 12 14, 16, 18, 20, 25, 30, 35, 40, 50, 60, 70, 80, 90, 100, 150, 200 or more). The fragment may comprise an epitope from an amino acid sequence from (a), (b), (c) or (d).

Preferred fragments are those disclosed in references 12 and 13. Other preferred fragments are C- and/or N-terminal truncations (*e.g.* Δ1-287, Δ2-287 *etc.).* Other preferred fragments may omit poly-glycine sequences from the full-length sequence. This has been found to aid expression [ref. 8]. Poly-glycine sequences can be represented as (Gly)*_{g}*, where *g*≥3 (e.g. 4, 5, 6, 7, 8, 9 or more). If a -X-moiety in (h) includes a poly-glycine sequence in its wild-type form, it is preferred to omit this sequence in the hybrid proteins of the invention. This may be by disrupting or removing the (Gly)*_{g}* - by deletion (*e*.*g.* CGGGGS→ CGGGS, CGGS, CGS or CS), by substitution (*e.g.* CGGGGS→ CGXGGS, CGXXGS, CGXGXS *etc.),* and/or by insertion (*e.g.* CGGGGS→ CGGXGGS, CGXGGGS, *etc.).* Deletion of (Gly)*_{g}* is preferred, and this deletion is referred to herein as 'ΔG', particularly deletion of the whole N-terminus up to and including the (Gly)*_{g}*. Poly-glycine omission is particularly useful for proteins 287, 741, 983 and Tbp2 (ΔG287, ΔG741, ΔG983 and ΔGTbp2 [8]).

Other preferred fragments may omit a leader peptide sequence from the full-length wild-type protein. This is particularly useful for proteins in group (h). In preferred proteins of group (h), all leader peptides in -X- moieties may be deleted except for that of the -X- moiety which is located at the N-terminus *i.e.* the leader peptide of X₁ will be retained, but the leader peptides of X₂ ... Xₙ will be omitted. This is equivalent to deleting all leader peptides and using the leader peptide of X₁ as moiety -A-.

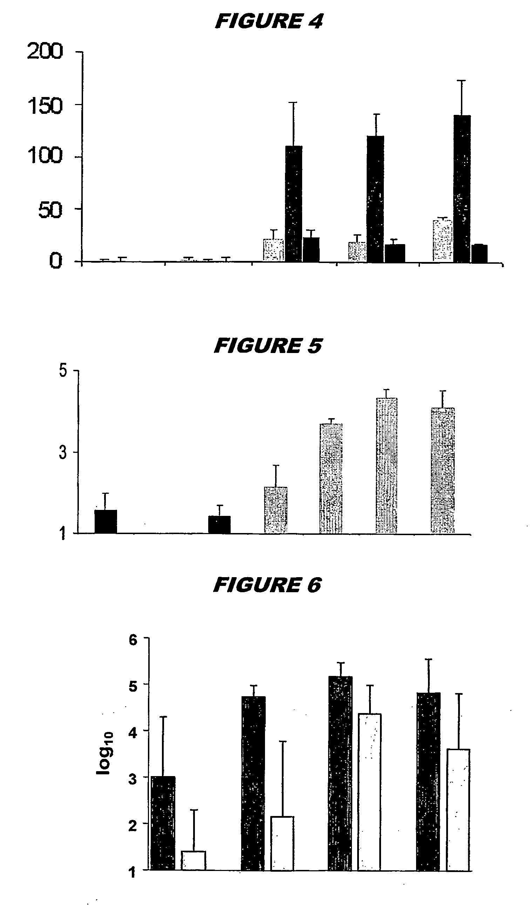

Other preferred fragments may omit complete protein domains. This is particularly useful for protein 961 ('NadA'), 287, and ORF46.1. Once a protein has been notionally divided into domains, (c) and (j) fragments can omit one or more of these domains (*e*.*g*. 287B, 287C, 287BC, ORF46₁₋₄₃₃, ORF46₄₃₃₋₆₀₈, ORF46, 961c - reference 8; Figures 8 and 9 in reference 9). 287 protein has been notionally split into three domains, referred to as A, B & C (see Figure 5 of reference 8). Domain B aligns strongly with IgA proteases, domain C aligns strongly with transferrin-binding proteins, and domain A shows no strong alignment with database sequences. An alignment of polymorphic forms of 287 is disclosed in reference 11. ORF46.1 has been notionally split into two domains - a first domain (amino acids 1-433) which is well-conserved between species and serogroups, and a second domain (amino acids 433-608) which is not well-conserved. The second domain is preferably deleted. An alignment of polymorphic forms of ORF46.1 is disclosed in reference 11. 961 protein has been notionally split into several domains (Figure 8 of reference 9).

Particularly preferred proteins in groups (a) to (d) comprise the amino acid sequence of (using the nomenclatures of references 1 to 9): orf1, orf4, orf25, orf40, orf46.1, orf83, NMB1343, 230, 233, 287, 292, 594, 687, 736, 741, 907, 919, 936, 953, 961 or 983. A preferred subset of these is: orf46.1, 230, 287, 741, 919, 936, 953, 961 and 983. A more preferred subset is: orf46.1, 287, 741 and 961.

**ORF1 (SEQ ID NO: 650 from** WO199924578**)**

MKTTDKRTTETHRKAPKTGRIRFSPAYLAICLSFGILPQAWAGHTYFGINYQY
YRDFAENKGKFAVGAKDIEVYNKKGELVGKSMTKAPMIDFSVVSRNGVAAL
VGDQYIVSVAHNGGYNNVDFGAEGRNPDQHRFTYKIVKRNNYKAGTKGHP
YGGDYHMPRLHKFVTDAEPVEMTSYMDGRKYIDQNNYPDRVRIGAGRQYW
RSDEDEPNNRESSYHIASAYSWLVGGNTFAQNGSGGGTVNLGSEKIKHSPYG
FLPTGGSFGDSGSPMFIYDAQKQKWLINGVLQTGNPYIGKSNGFQLVRKDWF
YDEIFAGDTHSVFYEPRQNGKYSFNDDNNGTGKINAKHEHNSLPNRLKTRTV
QLFNVSLSETAREPVYHAAGGVNSYRPRLNNGENISFIDEGKGELILTSNINQG
AGGLYFQGDFTVSPENNETWQGAGVHISEDSTVTWKVNGVANDRLSKIGKG
TLHVQAKGENQGSISVGDGTVILDQQADDKGKKQAFSEIGLVSGRGTVQLNA
DNQFNPDKLYFGFRGGRLDLNGHSLSFHRIQNTDEGAMIVNHNQDKESTVTI
TGNKDIATTGNNNSLDSKKEIAYNGWFGEKDTTKTNGRLNLVYQPAAEDRT
LLLSGGTNLNGNITQTNGKLFFSGRPTPHAYNHLNDHWSQKEGIPRGEIVWD
NDWINRTFKAENFQIKGGQAVVSRNVAKVKGDWHLSNHAQAVFGVAPHQS
HTICTRSDWTGLTNCVEKTITDDKVIASLTKTDISGNVDLADHAHLNLTGLAT
LNGNLSANGDTRYTVSHNATQNGNLSLVGNAQATFNQATLNGNTSASGNAS
FNLSDHAVQNGSLTLSGNAKANVSHSALNGNVSLADKAVFHFESSRFTGQIS
GGKDTALHLKDSEWTLPSGTELGNLNLDNATITLNSAYRHDAAGAQTGSAT
DAPRRRSRRSRRSLLSVTPPTSVESRFNTLTVNGKLNGQGTFRFMSELFGYRS
DKLKLAESSEGTYTLAVNNTGNEPASLEQLTVVEGKDNKPLSENLNFTLQNE
HVDAGAWRYQLIRKDGEFRLHNPVKEQELSDKLGKAEAKKQAEKDNAQSL
DALIAAGRDAVEKTESVAEPARQAGGENVGIMQAEEEKKRVQADKDTALAK
QREAETRPATTAFPRARRARRDLPQLQPQPQPQPQRDLISRYANSGLSEFSAT
LNSVFAVQDELDRVFAEDRRNAVWTSGIRDTKHYRSQDFRAYRQQTDLRQI
GMQKNLGSGRVGILFSHNRTENTFDDGIGNSARLAHGAVFGQYGIDRFYIGIS
AGAGFSSGSLSDGIGGKIRRRVLHYGIQARYRAGFGGFGIEPHIGATRYFVQK
ADYRYENVNIATPGLAFNRYRAGIKADYSFKPAQHISITPYLSLSYTDAASGK
VRTRVNTAVLAQDFGKTRSAEWGVNAEIKGFTLSLHAAAAKGPQLEAQHSA
GIKLGYRW

In relation to group (h), the value of *n* may be between 2 and *x*, and the value of *x* is typically 3, 4, 5, 6, 7, 8, 9 or 10. Preferably *n* is 2, 3 or 4; it is more preferably 2 or 3; most preferably, *n* = 2. Each -X-moiety is an amino acid sequence as specified in (a), (b), (c) or (d).

When n=2, preferred pairs of -X- moieties are: ΔG287 and 230; ΔG287 and 936; ΔG287 and 741; 961c and 287; 961c and 230; 961c and 936; 961cL and 287; 961cL and 230; 961cL and 936; ORF46.1 and 936; ORF46.1 and 230; 230 and 961; 230 and 741; 936 and 961; 936 and 741; ΔG741 and 741; ΔG287 and 287. Particularly preferred proteins are disclosed in references 14 and 15.

Where 287 is used in full-length form, it is preferably the final -Xₙ- moiety; if it is to be used at the N-terminus (*i*.*e*. as -X₁-), it is preferred to use a ΔG form of 287.

For each n instances of [-X-L-], linker amino acid sequence -L- may be present or absent. For instance, when n=2 the hybrid may be NH₂-X₁-L₁-X₂-L₂-COOH, NH₂-X₁-X₂-COOH, NH₂-X₁-L₁-X₂-COOH, NH₂-X₁-X₂-L₂-COOH, *etc*.

Linker amino acid sequence(s) -L- will typically be short (*e.g.* 20 or fewer amino acids *i.e.* 19, 18, 17, 16, 15, 14, 13, 12, 11, 10, 9, 8, 7, 6, 5, 4, 3, 2, 1). Examples include short peptide sequences which facilitate cloning, poly-glycine linkers (*i.e.* Gly*ₙ* where n = 2, 3, 4, 5, 6, 7, 8, 9, 10 or more), and histidine tags (*i*.*e*. His*ₙ* where n = 3, 4, 5, 6, 7, 8, 9, 10 or more). Other suitable linker amino acid sequences will be apparent to those skilled in the art.

If Xₙ₊₁ is a ΔG protein and Lₙ is a glycine linker, this may be equivalent to Xₙ₊₁ not being a ΔG protein and Lₙ being absent.

-A- is an optional N-terminal amino acid sequence. This will typically be short (*e*.*g*. 40 or fewer amino acids *i.e.* 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1). Examples include leader sequences to direct protein trafficking, or short peptide sequences which facilitate cloning or purification (*e*.*g*. histidine tags *i*.*e*. His*ₙ* where *n* = 3, 4, 5, 6, 7, 8, 9, 10 or more). Other suitable N-terminal amino acid sequences will be apparent to those skilled in the art.

-B- is an optional C-terminal amino acid sequence. This will typically be short (*e*.*g*. 40 or fewer amino acids *i.e.* 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1). Examples include sequences to direct protein trafficking, short peptide sequences which facilitate cloning or purification (*e*.*g*. histidine tags *i.e.* His*ₙ* where *n* = 3, 4, 5, 6, 7, 8, 9, 10 or more), or sequences which enhance protein stability. Other suitable C-terminal amino acid sequences will be apparent to those skilled in the art.

Amino acid sequences from any strain of *N.meningitidis* may be used. References to a particular protein (*e*.*g*. '287', or 'ORF46.1') therefore include that protein from any strain. Sequence variations between strains are included within (e) and (f).

Prototype sequences from *N.meningitidis* serogroup B include:

| **Protein** | **Prototype** | | **Protein** | **Prototype** |
|---|---|---|---|---|
| orf1 | Ref. 1, SEQ ID 650 | | orf4 | Ref. 1, SEQ ID 218 |
| orf25 | Ref. 1, SEQ ID 684 | | orf40 | Ref. 2, SEQ ID 4 |
| orf46.1 | Ref. 8, Example 8 | | orf83 | Ref. 1, SEQ ID 314 |
| NMB 1343 | Ref. 5, NMB 1343 | | 230 | Ref. 3, SEQ ID 830 |
| 233 | Ref. 3, SEQ ID 860 | | 287 | Ref. 3, SEQ ID 3104 |
| 292 | Ref. 3, SEQ ID 1220 | | 594 | Ref. 3, SEQ ID 1862 |
| 687 | Ref. 3, SEQ ID 2282 | | 736 | Ref. 3, SEQ ID 2506 |
| 741 | Ref. 3, SEQ ID 2536 | | 907 | Ref. 3, SEQ ID 2732 |
| 919 | Ref. 3, SEQ ID 3070 | | 936 | Ref. 3, SEQ ID 2884 |
| 953 | Ref. 3, SEQ ID 2918 | | 961 | Ref. 3, SEQ ID 940 |
| 983 | Ref. 5, NMB1969 | | | |

Reference 11 discloses polymorphic forms of proteins ORF4, ORF40, ORF46, 225, 235, 287, 519, 726, 919 and 953. Polymorphic forms of 961 are disclosed in references 16 and 17. Reference 9 discloses polymorphic forms of 741, 961 and NMB1343. Reference 15 discloses polymorphic forms of 741. Any of these polymorphic forms may be used.

Neisserial protein antigens are expressed in *Neisseria*, but the invention preferably utilises a heterologous host to express the antigen. The heterologous host may be prokaryotic (*e*.*g*. a bacterium) or eukaryotic. It is preferably *E.coli,* but other suitable hosts include *Bacillus subtilis, Vibrio cholerae, Salmonella typhi, Salmonenna typhimurium, Neisseria lactamica, Neisseria cinerea, Mycobacteria* (*e.g. Tuberculosis),* yeast, *etc.*

### The detoxified ADP-ribosylating toxin

ADP-ribosylating bacterial exotoxins are widely known. Examples include diphtheria toxin *(Corynebacterium diphtheriae),* exotoxin A (*Pseudomonas aeruginosa*), cholera toxin (CT; *Vibrio cholerae*), heat-labile enterotoxin (LT; *E.coli*) and pertussis toxin (PT).

The toxins catalyse the transfer of an ADP-ribose unit from NAD⁺ to a target protein.

The toxins are typically divided into two functionally distinct domains - A and B. The A subunit is responsible for the toxic enzymatic activity, whereas the B subunit is responsible for cellular binding. The subunits might be domains on the same polypeptide chain, or might be separate polypeptide chains. The subunits may themselves be oligomers *e*.*g*. the A subunit of CT consists of A₁ and A₂ which are linked by a disulphide bond, and its B subunit is a homopentamer. Typically, initial contact with a target cell is mediated by the B subunit and then subunit A alone enters the cell.

The toxins are typically immunogenic, but their inclusion in vaccines is hampered by their toxicity. To remove toxicity without also removing immunogenicity, the toxins have been treated with chemicals such as glutaraldehyde or formaldehyde. A more rational approach relies on site-directed mutagenesis of key active site residues to remove toxic enzymatic activity whilst retaining immunogenicity [*e.g*. refs. 18 (CT and LT), 19 (PT), 20 *etc*.]. Current acellular whooping cough vaccines include a form of pertussis toxin with two amino acid substitutions (Arg⁹→Lys and Glu¹²⁹→Gly; 'PT-9K/129G' [21]).

As well as their immunogenic properties, the toxins have been used as adjuvants. Parenteral adjuvanticity was first observed in 1972 [22] and mucosal adjuvanticity in 1984 [23]. It was surprisingly found in 1993 that the detoxified forms of the toxins retain adjuvanticity [24].

The compositions may include a detoxified ADP-ribosylating toxin. The toxin may be diphtheria toxin, *Pseudomonas* exotoxin A or pertussis toxin, but is preferably cholera toxin (CT) or, more preferably, *E.coli* heat-labile enterotoxin (LT). Other toxins which can be used are those disclosed in reference 25 (SEQ IDs 1 to 7 therein).

Detoxification of these toxins without loss of immunogenic and/or adjuvant activity can be achieved by any suitable means, with mutagenesis being preferred. Mutagenesis may involve one or more substitutions, deletions and/or insertions.

Preferred detoxified mutants are LT having a mutation at residue Arg-7 (*e.g*. a Lys substitution); CT having a mutation at residue Arg-7 (*e*.*g*. a Lys substitution); CT having a mutation at residue Arg-11 (*e*.*g*. a Lys substitution); LT having a mutation at Val-53; CT having a mutation at Val-53; CT having a mutation at residue Ser-61 (*e*.*g*. a Phe substitution); LT having a mutation at residue Ser-63 (*e.g.* a Lys or Tyr substitution) [*e*.*g*. Chapter 5 of ref. 26 - K63]; CT having a mutation at residue Ser-63 (*e.g.* a Lys or Tyr substitution); LT having a mutation at residue Ala-72 (*e.g.* an Arg substitution) [27 - R72]; LT having a mutation at Val-97; CT having a mutation at Val-97; LT having a mutation at Tyr-104; CT having a mutation at Tyr-104; LT having a mutation at residue Pro-106 (*e.g.* a Ser substitution); CT having a mutation at residue Pro-106 (*e.g.* a Ser substitution); LT having a mutation at Glu-112 (*e*.*g*. a Lys substitution); CT having a mutation at Glu-112 (*e*.*g*. a Lys substitution); LT having a mutation at residue Arg-192 (*e*.*g*. a Gly substitution); PT having a mutation at residue Arg-9 (*e.g.* a Lys substitution); PT having a mutation at Glu-129 (*e.g.* a Gly substitution); and any of the mutants disclosed in reference 18.

These mutations may be combined *e.g*. Arg-9-Lys + Glu-129-Gly in PT.

LT with a mutation at residue 63 or 72 is a preferred detoxified toxin.

The composition of the invention includes LT with a mutation at residue 72.

It will be appreciated that the numbering of these residues is based on prototype sequences and that, for example, although Ser-63 may not actually be the 63rd amino acid in a given LT variant, an alignment of amino acid sequences will reveal the location corresponding to Ser-63.

The detoxified toxins may be in the form of A and/or B subunits as appropriate for activity.

### Mucosal administration

The composition of the invention is particularly suited to mucosal immunisation, although parenteral immunisation is also possible. Suitable routes of mucosal administration include oral, intranasal, intragastric, pulmonary, intestinal, rectal, ocular, and vaginal routes. Oral or intranasal administration is preferred.

The composition is preferably adapted for mucosal administration [*e*.*g*. see refs. 28, 29 & 30]. Where the composition is for oral administration, for instance, it may be in the form of tablets or capsules (optionally enteric-coated), liquid, transgenic plants *etc.* [see also ref. 31, and Chapter 17 of ref. 32].

Where the composition is for intranasal administration, it may be in the form of a nasal spray, nasal drops, gel or powder *etc* [*e.g.* ref. 33].

### Further components of the composition

The composition may, in addition to the antigen and toxin components mentioned above, comprise one or more 'pharmaceutically acceptable carriers', which include any carrier that does not itself induce the production of antibodies harmful to the individual receiving the composition. Suitable carriers are typically large, slowly metabolised macromolecules such as proteins, polysaccharides, polylactic acids, polyglycolic acids, polymeric amino acids, amino acid copolymers, trehalose and lipid aggregates (such as oil droplets or liposomes). Such carriers are well known to those of ordinary skill in the art. The vaccines may also contain diluents, such as water, saline, glycerol, *etc.* Additionally, auxiliary substances, such as wetting or emulsifying agents, pH buffering substances, and the like, may be present. A thorough discussion of pharmaceutically acceptable excipients is available in *Remington's Pharmaceutical Sciences.*

Immunogenic compositions used as vaccines comprise an immunologically effective amount of antigen, as well as any other of the above-mentioned components, as needed. By 'immunologically effective amount', it is meant that the administration of that amount to an individual, either in a single dose or as part of a series, is effective for treatment or prevention. This amount varies depending upon the health and physical condition of the individual to be treated, age, the taxonomic group of individual to be treated (*e*.*g*. non-human primate, primate, *etc.),* the capacity of the individual's immune system to synthesise antibodies, the degree of protection desired, the formulation of the vaccine, the treating doctor's assessment of the medical situation, and other relevant factors. It is expected that the amount will fall in a relatively broad range that can be determined through routine trials. Dosage treatment may be a single dose schedule or a multiple dose schedule (*e.g*. including booster doses). The vaccine may be administered in conjunction with other immunoregulatory agents.

The composition may include other adjuvants in addition to detoxified toxin. Preferred adjuvants to enhance effectiveness of the composition include, but are not limited to: (1) oil-in-water emulsion formulations (with or without other specific immunostimulating agents such as muramyl peptides (see below) or bacterial cell wall components), such as for example (a) MF59™ (WO90/14837; Chapter 10 in ref. 32), containing 5% Squalene, 0.5% Tween 80, and 0.5% Span 85 (optionally containing MTP-PE) formulated into submicron particles using a microfluidizer, (b) SAF, containing 10% Squalane, 0.4% Tween 80, 5% pluronic-blocked polymer L121, and thr-MDP either microfluidized into a submicron emulsion or vortexed to generate a larger particle size emulsion, and (c) Ribi^{™} adjuvant system (RAS), (Ribi Immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox^{™}); (2) saponin adjuvants, such as QS21 or Stimulon^{™} (Cambridge Bioscience, Worcester, MA) may be used or particles generated therefrom such as ISCOMs (immunostimulating complexes), which ISCOMS may be devoid of additional detergent *e.g*. WO00/07621; (3) Complete Freund's Adjuvant (CFA) and Incomplete Freund's Adjuvant (IFA); (4) cytokines, such as interleukins (*e.g.* IL-1, IL-2, IL-4, IL-5, IL-6, IL-7, IL-12 (WO99/44636), *etc.),* interferons (*e*.*g*. gamma interferon), macrophage colony stimulating factor (M-CSF), tumor necrosis factor (TNF), *etc*.; (5) monophosphoryl lipid A (MPL) or 3-O-deacylated MPL (3dMPL) *e*.*g.* GB-2220221, EP-A-0689454; (6) combinations of 3dMPL with, for example, QS21 and/or oil-in-water emulsions *e*.*g*. EP-A-0835318, EP-A-0735898, EP-A-0761231; (7) oligonucleotides comprising CpG motifs [Krieg Vaccine 2000, 19, 618-622; Krieg Curn opin Mol They 2001 3:15-24; Roman et al., Nat. Med., 1997, 3, 849-854; Weiner et al., PNAS USA, 1997, 94, 10833-10837; Davis et al., J. Immunol., 1998, 160, 870-876; Chu et al., J. Exp. Med., 1997, 186, 1623-1631; Lipford et al., Eur. J. Immunol., 1997, 27, 2340-2344; Moldoveanu et al., Vaccine, 1988, 16, 1216-1224, Krieg et al., Nature, 1995, 374, 546-549; Klinman et al., PNAS USA, 1996, 93, 2879-2883; Ballas et al., J. Immunol., 1996, 157, 1840-1845; Cowdery et al., J. Immunol., 1996, 156, 4570-4575; Halpern et al., Cell. Immunol., 1996, 167, 72-78; Yamamoto et al., Jpn. J. Cancer Res., 1988, 79, 866-873; Stacey et al., J. Immunol., 1996, 157, 2116-2122; Messina et al., J. Immunol., 1991, 147, 1759-1764; Yi et al., J. Immunol., 1996, 157, 4918-4925; Yi et al., J. Immunol., 1996, 157, 5394-5402; Yi et al., J. Immunol., 1998, 160, 4755-4761; and Yi et al., J. Immunol., 1998, 160, 5898-5906; International patent applications WO96/02555, WO98/16247, WO98/18810, WO98/40100, WO98/55495, WO98/37919 and WO98/52581] *i*.*e*. containing at least one CG dinucleotide, with 5-methylcytosine optionally being used in place of cytosine; (8) a polyoxyethylene ether or a polyoxyethylene ester *e.g*. WO99/52549; (9) a polyoxyethylene sorbitan ester surfactant in combination with an octoxynol (*e*.*g*. WO01/21207) or a polyoxyethylene alkyl ether or ester surfactant in combination with at least one additional non-ionic surfactant such as an octoxynol (*e.g.* WO01/21152); (10) an immunostimulatory oligonucleotide (*e.g.* a CpG oligonucleotide) and a saponin *e.g*. WO00/62800; (11) an immunostimulant and a particle of metal salt *e*.*g.* WO00/23105; (12) a saponin and an oil-in-water emulsion *e.g.* WO99/11241; (13) a saponin (*e.g.* QS21) + 3dMPL + IL-12 (optionally + a sterol) *e*.*g*. WO98/57659; (14) PLG microparticles; (15) other substances that act as immunostimulating agents to enhance the efficacy of the composition.

Muramyl peptides include N-acetyl-muramyl-L-threonyl-D-isoglutamine (thr-MDP), N-acetyl-normuramyl-L-alanyl-D-isoglutamine (nor-MDP), N-acetylmuramyl-L-alanyl-D-isoglutaminyl-L-alanine-2-(1'-2'-dipalmitoyl-*sn*-glycero-3-hydroxyphosphoryloxy)-ethylamine MTP-PE), *etc.*

Compositions may comprise a buffer. Compositions may be buffered at between pH 6 and pH 8 (*e*.*g*. at about pH 7).

Compositions may be sterile and/or pyrogen-free.

### Further antigens

Further antigens which can be included in the composition includes:
- an outer-membrane vesicle (OMV) preparation from *N.meningitidis* serogroup B, such as those disclosed in refs. 34, 35, 36, 37 *etc.*
- a saccharide antigen from *N.meningitidis* serogroup A, C, W135 and/or Y, such as the oligosaccharide disclosed in ref. 38 from serogroup C [see also ref. 39].
- a saccharide antigen from *Streptococcus pneumoniae* [*e.g.* refs. 40, 41, 42].
- a protein antigen from *Helicobacter pylori* such as CagA [*e.g.* 43], VacA [*e.g.* 43], NAP [*e.g.* 44], HopX [*e.g.* 45], HopY [*e.g.* 45] and/or urease.
- an antigen from hepatitis A virus, such as inactivated virus [*e*.*g*. 46, 47].
- an antigen from hepatitis B virus, such as the surface and/or core antigens [*e*.*g*. 47, 48].
- an antigen from hepatitis C virus [*e*.*g*. 49].
- an antigen from *B*o*rdetella pertussis,* such as pertussis holotoxin (PT) and filamentous haemagglutinin (FHA) from *B.pertussis,* optionally also in combination with pertactin and/or agglutinogens 2 and 3 [*e*.*g*. refs. 50 & 51].
- a diphtheria antigen, such as a diphtheria toxoid [*e*.*g*. chapter 3 of ref. 52] *e*.*g*. the CRM₁₉₇ mutant [*e*.*g*. 26].
- a tetanus antigen, such as a tetanus toxoid [*e*.*g*. chapter 4 of ref. 52].
- a saccharide antigen from *Haemophilus influenzae* B [*e.g*. 39].
- an antigen from *N.gonorrhoeae* [*e.g.* 1, 2, 3].
- an antigen from *Chlamydia pneumoniae* [*e.g.* 53, 54, 55, 56, 57, 58, 59].
- an antigen from *Chlamydia trachomatis* [*e.g.* 60].
- an antigen from *Porphyromonas gingivalis* [*e.g*. 61].
- polio antigen(s) [*e*.*g*. 62, 63] such as IPV or OPV.
- rabies antigen(s) [*e*.*g*. 64] such as lyophilised inactivated virus [e.g.65, RabAvert^{™}].
- measles, mumps and/or rubella antigens [*e*.*g*. chapters 9, 10 & 11 of ref. 52].
- influenza antigen(s) [*e*.*g*. chapter 19 of ref. 52], such as the haemagglutinin and/or neuraminidase surface proteins.
- an antigen from *Moraxella catarrhalis* [*e.g*. 66].
- an antigen from *Streptococcus agalactiae* (group B streptococcus) [*e*.*g*. 67, 68].
- an antigen from *Streptococcus pyogenes* (group A streptococcus) [*e*.*g*. 68, 69, 70].
- an antigen from *Staphylococcus aureus* [*e.g.* 71].

The composition may comprise one or more of these further antigens.

Where a saccharide or carbohydrate antigen is used, it is preferably conjugated to a carrier protein in order to enhance immunogenicity [*e*.*g*. refs. 72 to 81]. Preferred carrier proteins are bacterial toxins or toxoids, such as diphtheria or tetanus toxoids. The CRM₁₉₇ diphtheria toxoid is particularly preferred. Other suitable carrier proteins include the *N.meningitidis* outer membrane protein [*e.g.* ref. 82], synthetic peptides [*e.g.* 83, 84], heat shock proteins [*e.g.* 85], pertussis proteins [*e.g.* 86, 87], protein D from *H.influenzae* [*e.g.* 88], toxin A or B from *C.difficile* [*e.g.* 89], *etc.* Where a mixture comprises capsular saccharides from both serogroups A and C, it is preferred that the ratio (w/w) of MenA saccharide:MenC saccharide is greater than 1 (*e.g.* 2:1, 3:1, 4:1, 5:1, 10:1 or higher). Saccharides from different serogroups of *N.meningitidis* may be conjugated to the same or different carrier proteins.

Any suitable conjugation reaction can be used, with any suitable linker where necessary.

Toxic protein antigens may be detoxified where necessary (*e*.*g*. detoxification of pertussis toxin by chemical and/or genetic means [51]).

Where a diphtheria antigen is included in the composition it is preferred also to include tetanus antigen and pertussis antigens. Similarly, where a tetanus antigen is included it is preferred also to include diphtheria and pertussis antigens. Similarly, where a pertussis antigen is included it is preferred also to include diphtheria and tetanus antigens.

Antigens are preferably mixed with (and more preferably adsorbed to) an aluminium salt (*e.g.* phosphate, hydroxide, hydroxyphosphate, oxyhydroxide, orthophosphate, sulphate). The salt may take any suitable form (*e.g.* gel, crystalline, amorphous *etc.*).

Antigens in the composition will typically be present at a concentration of at least 1µg/ml each. In general, the concentration of any given antigen will be sufficient to elicit an immune response against that antigen.

As an alternative to using proteins antigens in the composition of the invention, nucleic acid encoding the antigen may be used [*e*.*g*. refs. 90 to 98]. Protein components of the compositions of the invention may thus be replaced by nucleic acid (preferably DNA *e.g*. in the form of a plasmid) that encodes the protein. Such nucleic acid can be prepared in many ways *(eg.* by chemical synthesis, from genomic or cDNA libraries, from the organism itself *etc.)* and can take various forms *(eg.* single stranded, double stranded, vectors, probes *etc.).* The term "nucleic acid" includes DNA and RNA, and also their analogues, such as those containing modified backbones, and also peptide nucleic acids (PNA) *etc.*

### Medicaments

The composition of the invention is typically a vaccine composition.

The invention provides the compositions defined above for use as medicaments. The medicament is preferably able to raise an immune response in a mammal against the antigen (*i*.*e*. it is an immunogenic composition) and is more preferably a vaccine.

The invention provides the use of the compositions defined above in the manufacture of a medicament for treating or preventing infection due to *Neisseria* bacteria (preferably *N.meningitidis*).

There is described a method of raising an immune response in an animal (*e*.*g*. a mammal, such as a mouse or a human), comprising administering to the animal a composition of the invention. The immune response is preferably protective. The animal is preferably 0-3 years old.

There is described a method of treating a patient, comprising administering to the patient a therapeutically effective amount of a composition of the invention.

Vaccines according to the invention may either be prophylactic (*i*.*e*. to prevent infection) or therapeutic (*i*.*e*. to treat disease after infection), but will typically be prophylactic.

These uses and methods *etc.* are preferably for the prevention and/or treatment of a disease caused by a *Neisseria* (*e*.*g.* meningitis, septicaemia, gonorrhoea *etc.).* The prevention and/or treatment of bacterial meningitis is preferred.

The efficacy of an immunogenic composition can be assessed by monitoring antigen-specific immune responses (*e*.*g*. T cell or antibody responses) raised in an animal following administration of the composition.

The generation of bactericidal antibodies in an animal following administration of a composition of the invention is also indicative of efficacy.

### Definitions

The term "comprising" means "including" as well as "consisting" *e.g*. a composition "comprising" X may consist exclusively of X or may include something additional *e.g*. X + Y.

The term "about" in relation to a numerical value x means, for example, *x*±10%.

### BRIEF DESCRIPTION OF DRAWINGS

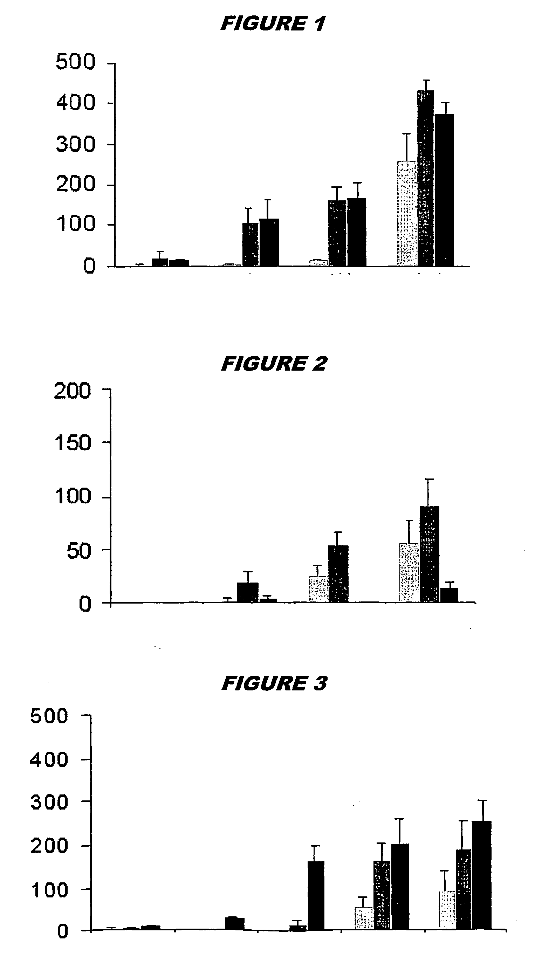

Figures 1 to 4 show IFN-γ (Figures 1 & 3; ng/ml) and IL-5 (Figures 2 and 4; pg/ml) levels induced by orf1 (Figures 1 & 2) and orf40 (figures 3 and 4). For figures 1 & 2, there are four data sets on the X-axis (left to right: PBS; orf1; orf1 + 1µg/ml LT-K63; orf1 + 1µg/ml LT-R72), each of which has three values for different antigen concentrations (left to right: 0.1µg/ml; 10µg/ml; 100µg/ml). For figures 3 & 4, there are five data sets on the X-axis (left to right: PBS; orf40; orf40 + 1µg/ml LT-K63; orf40 + 10µg/ml LT-K63; orf40 + 1µg/ml LT-R72), each of which has three values for different antigen concentrations (left to right: 0.1 µg/ml; 10µg/ml; 100µg/ml).
Figure 5 shows IgG titres (log₁₀) induced by orf1 and orf40. The seven columns are, from left to right: orf1; orf1 + 1µg/ml LT-K63; 1µg/ml LT-R72; orf40; orf40 + 1µg/ml LT-K63; orf40 + 10µg/ml LT-K63; orf40 + 1µg/ml LT-R72.
Figure 6 shows the IgG antibody subclass responses against ORF40. The four data sets are the same as the right-hand four sets in Figure 5. In each data set, the left-hand column shows IgG1 and the right-hand column shows IgG2a.

### MODES FOR CARRYING OUT THE INVENTION

### Intranasal immunisation with N.meningitidis protein antigens

Groups of five female Balb/c mice were immunised intranasally under ether anaesthesia on days 0, 21 and 42 with compositions comprising: (a) *N.meningitidis* (serogroup B) antigen orf1 or orf40; and (b) *E.coli* heat label toxin mutant R72 or K63. Negative control mice received either PBS or antigen without LT adjuvant. The groups were as follows:

| **Group** | **Antigen** | **Adjuvant** |
|---|---|---|
| 1 | - | - |
| 2 | Orf1 | - |
| 3 | Orf1 | LT-K63 (1µg) |
| 4 | Orf1 | LT-R72 (1µg) |
| 5 | Orf40 | - |
| 6 | Orf40 | LT-K63 (1µg) |
| 7 | Orf40 | LT-K63 (10µg) |
| 8 | Orf40 | LT-R72 (1µg) |

Antigens (5µg/mouse) and adjuvants were delivered in a dose of 20µl per mouse.

Two weeks after the final immunisation, animals were sacrificed and blood, spleens and cervical lymph nodes were taken from mice. Sera was collected and stored for analysis of orfl/orf40-specific IgG, IgG1 and IgG2a by ELISA. Single cell suspensions were prepared from the spleens and cervical lymph nodes. The cells were set up in 96 well plates with orf1 or orf40 at 0, 0.1, 10 and 100µg/ml. As a positive control, the cells were incubated with PMA and anti-CD3. Cells were incubated at 37°C with 5% CO₂ for 3 days. On day 3, supernatants were collected for analysis of IL4, IL5 and interferon γ (IFNγ). ³H-thymidine was added to the cells and plates were further incubated for 4 hr to allow an estimation of antigen-specific proliferation (data not shown).

Re-stimulated splenocytes from mice immunised with orf1 produced IFNγ and small amounts of IL-5 (figures 1 & 2). When mice were immunised with orf1 and LTK63 and in particular with LTR72 the cytokine responses were considerably stronger.

In contrast, immunisation with the antigen alone or together with the LT mutants did not lead to the production of significant levels of specific antibody (figure 5).

Immunisation with orf40 alone elicited a specific antibody response (figure 5). This was mainly of the IgG1 subclass (figure 6). Re-stimulated splenocytes from these mice did not produce significant amounts of IL-5 or IFNγ (figures 3 & 4). Immunisation with orf40 and LTK63 (1µg) resulted in a large increase in IL-5 production and increased IFNγ. Immunisation with orf40 and 10µg of LTK63 led to a higher IFNγ concentration in supernatants. Restimulated splenocytes from mice immunised with orf 40 and LTR72 (1µg) secreted IL-5 and IFNγ at concentrations comparable to those from mice immunised with the higher dose of LTK63. Sera from these mice contained high titres of specific IgG1 and IgG2a. In contrast, sera from mice immunised with orf40 and LTK63 contained higher titres of specific IgG1 than immunisation with the antigen alone. However, titres of specific IgG2a were not enhanced. Immunisation with the 10µg dose of LTK63 resulted in higher titres of specific IgG2a. This correlated with the higher production of IFNγ in this group. The individual titres of orf40-specific IgG1 and IgG2a are presented in Table 1. These will be required to set up the assays for bactericidal antibody titres.

**Table. 1. Individual IgG1 and IgG2a subclass titres from mice immunised with orf40 alone or together with LTK63/LTR72**

| **orf40 only** | | **orf40 + K63(1µg)** | | **orf40+K63(10µg)** | | **Orf40 + R72(1µg)** | |
|---|---|---|---|---|---|---|---|
| **IgG1** | **IgG2a** | **IgG1** | **IgG2a** | **IgG1** | **IgG2a** | **IgG1** | **IgG2a** |
| 1 | 1 | 25000 | 1 | 100000 | 2500 | 15000 | 100 |
| 600 | 1 | 40000 | 1 | 100000 | 15000 | 15000 | 900 |
| 1500 | 1 | 60000 | 1 | 100000 | 35000 | 50000 | 9000 |
| 4000 | 1 | 80000 | 2500 | 150000 | 70000 | 150000 | 10000 |
| 30000 | 1000 | 100000 | 25000 | 500000 | 80000 | 800000 | 150000 |

### REFERENCES

1 - International patent application WO99/24578.
2 - International patent application WO99/36544.
3 - International patent application WO99/57280.
4 - International patent application WO00/22430.
5 - Tettelin et al. (2000) Science 287:1809-1815.
6 - Pizza et al. (2000) Science 287:1816-1820.
7 - International patent application WO01/64920.
8 - International patent application WO01/64922.
9 - International patent application PCT/IB02/03904.
10 - International patent application WO00/50075.
11- International patent application WO00/66741.
12 - International patent application WO00/71574.
13 - International patent application WO01/04316.
14 - United Kingdom patent application 0223741.0.
15 - United Kingdom patent application 0227346.4.
16 - International patent application PCT/IB02/03396
[17] Comanducci et al. (2002) J. Exp. Med. 195:1445-1454.
18 - International patent application WO93/13202.
19 - European patent applications 0306618, 0322533 and 0322115.
20 - Del Giudice & Rappuoli (1999) Vaccine 1999 17 Suppl 2:S44-52
21 - European patent 0396964.
22 - Northrup & Fauci (1972) J. Infect. Dis. 125:672ff.
23 - Elson & Ealding (1984) J. Immunol. 133:2892ff and 132:2736ff.
24 - International patent application WO95/17211.
25 - International patent application WO02/079242.
26 - Del Giudice et al. (1998) Molecular Aspects of Medicine, vol. 19, number 1.
27 - International patent application WO98/18928.
28 - Walker (1994) Vaccine 12:387-400.
29 - Clements (1997) Nature Biotech. 15:622-623.
30 - McGhee et al. (1992) Vaccine 10:75-88.
31 - Michetti (1998) J. Gastroenterol. [Suppl X]:66-68.
32 - Vaccine design: the subunit and adjuvant approach, eds. Powell & Newman, Plenum Press 1995 (ISBN 0-306-44867-X.
33 - Almeida & Alpar (1996) J. Drug Targeting 3:455-467.
34 - International patent application WO01/52885.
35 - Bjune et al. (1991) Lancet 338(8775):1093-1096.
36 - Fukasawa et al. (1999) Vaccine 17:2951-2958.
37 - Rosenqvist et al. (1998) Dev. Biol. Stand. 92:323-333.
38 - Costantino et al. (1992) Vaccine 10:691-698.
39 - Costantino et al. (1999) Vaccine 17:1251-1263.
40 - Watson (2000) Pediatr Infect Dis J 19:331-332.
41 - Rubin (2000) Pediatr Clin North Am 47:269-285, v.
42 - Jedrzejas (2001) Microbiol Mol Biol Rev 65:187-207.
43 - International patent application WO93/18150.
44 - International patent application WO99/53310.
45 - International patent application WO98/04702.
46 - Bell (2000) Pediatr Infect Dis J 19:1187-1188.
47 - Iwarson (1995) APMIS 103:321-326.
48 - Gerlich et al. (1990) Vaccine 8 Suppl:S63-68 & 79-80.
49 - Hsu et al. (1999) Clin Liver Dis 3:901-915.
50 - Gustafsson et al. (1996) N. Engl. J. Med 334:349-355.
51 - Rappuoli et al. (1991) TIBTECH 9:232-238.
52 - Vaccines (1988) eds. Plotkin & Mortimer. ISBN 0-7216-1946-0.
53 - International patent application WO02/02606.
54 - Kalman et al. (1999) Nature Genetics 21:385-389.
55 - Read et al. (2000) Nucleic Acids Res 28:1397-406.
56 - Shirai et al. (2000) J. Infect. Dis. 181(Suppl 3):S524-S527.
57 - International patent application WO99/27105.
58 - International patent application WO00/27994.
59 - International patent application WO00/37494.
60 - International patent application WO99/28475.
61- Ross et al. (2001) Vaccine 19:4135-4142.
62 - Sutter et al. (2000) Pediatr Clin North Am 47:287-308.
63 -Zimmerman & Spann (1999) Am Fam Physician 59:113-118, 125-126.
64 - Dreesen (1997) Vaccine 15 Suppl:S2-6.
65 - MMWR Morb Mortal Wkly Rep 1998 Jan 16;47(1):12, 19.
66 - McMichael (2000) Vaccine 19 Suppl 1:S101-107.
67 - Schuchat (1999) Lancet 353(9146):51-6.
68 - International patent application WO02/34771.
69 - Dale (1999) Infect Dis Clin North Am 13:227-43, viii.
70 - Ferretti et al. (2001) PNAS USA 98: 4658-4663.
71 - Kuroda et al. (2001) Lancet 357(9264):1225-1240; see also pages 1218-1219.
72 - Ramsay et al. (2001) Lancet 357(9251):195-196.
73 -Lindberg (1999) Vaccine 17 Suppl 2:S28-36.
74 - Buttery & Moxon (2000) J R Coll Physicians Lond 34:163-168.
75 - Ahmad & Chapnick (1999) Infect Dis Clin North Am 13:113-133, vii.
76 - Goldblatt (1998) J. Med. Microbiol. 47:563-567.
77 - European patent 0 477 508.
78 - US patent 5,306,492.
79 - International patent application WO98/42721.
80 - Conjugate Vaccines (eds. Cruse et al.) ISBN 3805549326, particularly vol. 10:48-114.
81 -Hermanson (1996) Bioconjugate Techniques ISBN: 0123423368 or 012342335X.
82 - European patent application 0372501.
83 - European patent application 0378881.
84 - European patent application 0427347.
85 - International patent application WO93/17712.
86 - International patent application WO98/58668.
87 - European patent application 0471177.
88 - International patent application WO00/56360.
89 - International patent application WO00/61761.
90 - Robinson & Torres (1997) Seminars in Immunology 9:271-283.
*91 -*Donnelly et al. (1997) Annu Rev Immunol 15:617-648.
92 - Scott-Taylor & Dalgleish (2000) Expert Opin Investig Drugs 9:471-480.
93 - Apostolopoulos & Plebanski (2000) Curr Opin Mol Ther 2:441-447.
94 - Ilan (1999) Curr Opin Mol Ther 1:116-120.
95 - Dubensky et al. (2000) Mol Med 6:723-732.
96 - Robinson & Pertmer (2000) Adv Virus Res 55:1-74.
97 - Donnelly et al. (2000) Am J Respir Crit Care Med 162(4 Pt 2): S190-193.
98 - Davis (1999) Mt. Sinai J. Med. 66:84-90.

## Claims

1. A composition comprising:
(a) Neisseria meningitidis serogroup B antigen ORF40; and
(b) a detoxified ADP-ribosylating toxin, wherein said detoxified ADP-ribosylating toxin is *E.coli* heat-labile enterotoxin LT R72.

2. The composition of claim 1, wherein the composition is adapted for mucosal administration.

3. The composition of claim 2, wherein the composition is adapted for intranasal administration.

4. The composition of any preceding claim, wherein the composition further comprises one or more of the following antigens:
- an outer-membrane vesicle (OMV) preparation from *N.meningitidis*;
- a saccharide antigen from *N.meningitidis*;
- a saccharide antigen from *Streptococcus pneumoniae*;
- an antigen from hepatitis A, B or C virus;
- an antigen from *Bordetella pertussis*;
- a diphtheria antigen;
- a tetanus antigen;
- a protein antigen from *Helicobacter pylori*;
- a saccharide antigen from *Haemophilus influenzae*;
- an antigen from *N.gonorrhoeae*;
- an antigen from *Chlamydia pneumoniae*;
- an antigen from *Chlamydia trachomatis*;
- an antigen from *Porphyromonas gingivalis*;
- polio antigen(s);
- rabies antigen(s);
- measles, mumps and/or rubella antigens;
- influenza antigen(s);
- an antigen from *Moraxella catarrhalis*;
- an antigen from *Streptococcus agalactiae*;
- an antigen from *Streptococcus pyogenes*; and/or
- an antigen from *Staphylococcus aureus*.

5. The composition of any preceding claim, further comprising a buffer.

6. The composition of any preceding claim, having a pH between 6 and 8.

7. The composition of any preceding claim, wherein the composition is sterile and/or pyrogen-free.

8. The composition of any one of claims 1 to 7 for use as a medicament.

9. The use of the composition of any one of claims 1 to 7 in the manufacture of a medicament for treating or preventing infection due to *Neisseria* bacteria.

10. The composition of any one of claims 1 to 7, wherein the composition is an immunogenic composition.

11. The composition of any one of claims 1 to 7, wherein the composition is a vaccine.

## Patentansprüche

1. Zusammensetzung, umfassend:
(a) das ORF40-Antigen von *Neisseria meningitidis* der Serogruppe B; und
(b) ein detoxofiziertes ADP-ribosylierendes Toxin, wobei das detoxofizierte ADP-ribosylierende Toxin das hitzelabile Enterotoxin LT R72 von *E. coli* ist.

2. Zusammensetzung nach Anspruch 1, wobei die Zusammensetzung für die mukosale Verabreichung angepasst ist.

3. Zusammensetzung nach Anspruch 2, wobei die Zusammensetzung für die intranasale Verabreichung angepasst ist.

4. Zusammensetzung nach einem der vorhergehenden Ansprüche, wobei die Zusammensetzung ferner ein oder mehrere der folgenden Antigene umfasst:
- eine Zubereitung von Vesikeln der äußeren Membran (outer membrane vesicle, OMV) von *N. meningitidis;*
- ein Saccharid-Antigen von *N. meningitidis*;
- ein Saccharid-Antigen von *Streptococcus pneumoniae*;
- ein Antigen von Hepatitis A-, B- oder C-Virus
- ein Antigen von *Bordetella pertussis*;
- ein Diphtherie-Antigen;
- ein Tetanus-Antigen;
- ein Protein-Antigen von *Helicobacter pylori*;
- ein Saccharid-Antigen von *Haemophilus influenzae*;
- ein Antigen von *N. gonorrhoeae*;
- ein Antigen von *Chlamydia pneumoniae*;
- ein Antigen von *Chlamydia trachomatis*;
- ein Antigen von *Porphyromonas gingivalis*;
- Polio-Antigen(e);
- Tollwut-Antigen(e);
- Masern-, Mumps- und/oder Röteln-Antigene;
- Influenza-Antigen(e);
- ein Antigen von *Moraxella catarrhalis;*
- ein Antigen von *Streptococcus agalactiae;*
- ein Antigen von *Streptococcus pyogenes;* und/oder
- ein Antigen von *Staphylococcus aureus.*

5. Zusammensetzung nach einem der vorhergehenden Ansprüche, die ferner einen Puffer umfasst.

6. Zusammensetzung nach einem der vorhergehenden Ansprüche, die einen pH-Wert zwischen 6 und 8 aufweist.

7. Zusammensetzung nach einem der vorhergehenden Ansprüche, wobei die Zusammensetzung steril und/oder Pyrogen-frei ist.

8. Zusammensetzung nach einem der Ansprüche 1 bis 7 zur Verwendung als Medikament.

9. Verwendung einer Zusammensetzung nach einem der Ansprüche 1 bis 7 bei der Herstellung eines Medikaments zur Behandlung oder Vermeidung einer durch Neisseria-Bakterien verursachten Infektion.

10. Zusammensetzung nach einem der Ansprüche 1 bis 7, wobei die Zusammensetzung eine immunogene Zusammensetzung ist.

11. Zusammensetzung nach einem der Ansprüche 1 bis 7, wobei die Zusammensetzung ein Impfstoff ist.

## Revendications

1. Composition comprenant :
(a) l'antigène ORF40 de *Neisseria meningitidis* de sérogroupe B ; et
(b) une toxine ADP-ribosylante détoxifiée, ladite toxine ADP-ribosylante détoxifiée étant l'entérotoxine LT R72 thermolabile de *E. coli*.

2. Composition selon la revendication 1, la composition étant adaptée pour l'administration muqueuse.

3. Composition selon la revendication 2, la composition étant adaptée pour l'administration intra-nasale.

4. Composition selon l'une quelconque des revendications précédentes, ladite composition comprenant en outre un ou plusieurs des antigènes suivantes :
■ une préparation de vésicule de membrane externe (OMV) de *N. meningi tidis ;*
■ un antigène saccharidique de *N. meningitidis ;*
■ un antigène saccharidique de *Streptococcus pneumoniae ;*
■ un antigène du virus de l'hépatite A, B ou C ;
■ un antigène de *Bordetella pertussis ;*
■ un antigène diphtérique ;
■ un antigène tétanique ;
■ un antigène protéique de *Helicobacter pylori ;*
■ un antigène saccharidique de *Haemophilus influenzae ;*
■ un antigène de *N. gonorrhoeae* ;
■ un antigène de *Chlamydia pneumoniae* ;
■ un antigène de *Chlamydia trachomatis* ;
■ un antigène de *Porphyromonas gingivalis* ;
■ un/des antigène(s) de la poliomyélite ;
■ un/des antigènes de la rage ;
■ des antigènes de la rougeole, des oreillons et/ou de la rubéole ;
■ un/des antigène(s) de la grippe ;
■ un antigène de *Moraxella catarrhalis* ;
■ un antigène de *Streptococcus agalactiae* ;
■ un antigène de *Streptococcus pyogenes ;* et/ou
■ un antigène de *Staphylococcus aureus*.

5. Composition selon l'une quelconque des revendications précédentes, comprenant en outre un tampon.

6. Composition selon l'une quelconque des revendications précédentes, ayant un pH entre 6 et 8.

7. Composition selon l'une quelconque des revendications précédentes, la composition étant stérile et/ou dépourvue d'agents pyrogènes.

8. Composition selon l'une quelconque des revendications 1 à 7, pour une utilisation comme médicament.

9. Utilisation de la composition selon l'une quelconque des revendications 1 à 7, dans la production d'un médicament destiné à traiter ou à prévenir une infection due à la bactérie *Neisseria.*

10. Composition selon l'une quelconque des revendications 1 à 7, la composition étant une composition immunogène.

11. Composition selon l'une quelconque des revendications 1 à 7, la composition étant un vaccin.
